# FASCICULE DE BREVET EUROPEEN

(11) **EP 0 748 625 B1**
(45) Date de publication et mention de la délivrance du brevet: **30.12.1998**
(21) Numéro de dépôt: 96401191.0
(22) Date de dépôt: 05.06.1996
(51) Int. Cl.: A61K 7/48

(54) **Composition cosmétique ou dermatologique à libération contrôlée d'actif contenant un caroténoide photoconvertible**
Kosmetische oder dermatologische Zusammensetzung, mit kontrollierter Freisetzung des Wirkstoffes ein photoconvertibles Carotenoid enthaltend
Cosmetic or dermatologic compositions with controlled release of the active agent containing a photoconvertible carotenoid

(30) Priorité: 16.06.1995 FR 9507211
(43) Date de publication de la demande: 18.12.1996
(73) Titulaire: L'OREAL, 75008 Paris (FR)
(72) Inventeur: Soudant, Etienne, 94260 Fresnes (FR); Koulbanis, Constantin, 94270 Le Kremlin-Bicètre (FR)
(74) Mandataire: Casalonga, Axel

(56) Documents cités:
- EP-A- 0 467 795
- EP-A- 0 631 772
- FR-A- 2 100 886
- FR-A- 2 681 784
- US-A- 4 603 146

## Description

La présente invention concerne l'utilisation d'un caroténoïde photoconvertible pour protéger la peau contre le photovieillissement et pour la prévention de l'acné, ainsi qu'une composition cosmétique ou dermatologique à libération contrôlée d'actif contenant un caroténoïde photoconvertible.

L'acide rétinoïque a déjà été utilisé sous le nom de "vitamine A acide", par exemple dans la demande EP 230 498, dans des compositions destinées à lutter contre le photovieillissement de la peau.

Les rétinoïdes utilisés en dermatologie sont généralement l'acide rétinoïque et ses isomères et possèdent une réelle activité biologique. Cependant, ils sont pourvus d'effets secondaires importants, qu'ils soient utilisés par voie systémique ou par voie topique, et provoquent notamment une forte irritation de la peau.

C'est pourquoi, selon la demande de brevet français 2 681 784, on utilise en dermatologie, stomatologie ou cosmétologie, notamment pour le traitement d'affections telles que le psoriasis, l'acné, l'eczéma, etc..., pour le traitement des affections des muqueuses ou pour le traitement des troubles dûs au vieillissement et/ou de la séborrhée, des bioprécurseurs d'acide rétinoïque ou de rétinol qui, lorsqu'ils sont mis en contact avec des cellules épidermiques, entraînent la biosynthèse d'acide rétinoïque et/ou de rétinol par l'intermédiaire des systèmes enzymatiques propres à chaque cellule. Parmi ces bioprécurseurs, on peut citer en particulier le rétinal qui aboutit à la formation intracellulaire du rétinol et de l'acide rétinoïque.

La demanderesse a découvert de manière surprenante que les rétinoïdes utiles en cosmétologie et dermatologie tels que le rétinol et l'acide rétinoïque ainsi que ses isomères, pouvaient être libérés sur la peau de façon contrôlée par photoconversion de caroténoïdes par l'intermédiaire d'espèces activées hydrophiles de l'oxygène.

Une telle photoconversion peut se produire sous l'effet d'un "stress oxydant", c'est-à-dire toute agression atmosphérique externe génératrice d'espèces activées hydrophiles de l'oxygène. Un tel "stress oxydant" peut être la conséquence d'une irradiation UV ou encore de la pollution.

La présente invention a donc pour objet l'utilisation d'un caroténoïde photoconvertible de formule : 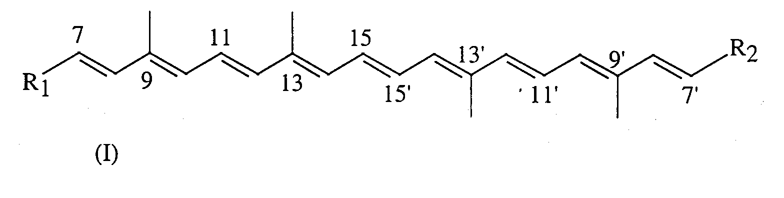 dans laquelle les substituants R₁ et R₂ désignent l'un des groupements suivants : 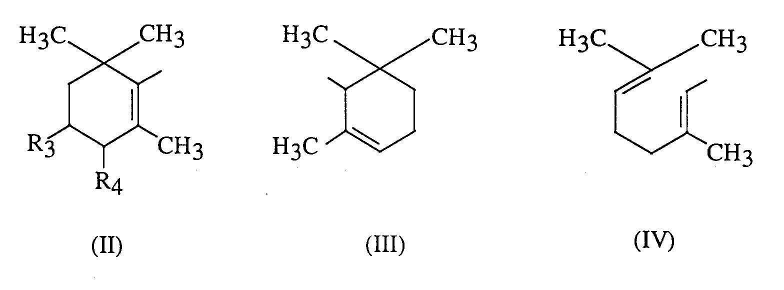 l'un au moins des substituants R₁ ou R₂ désignant un groupement β-ionone de formule (II),
les substituants R₃ et R₄ désignant de l'hydrogène, un groupe hydroxy, carboxy, alcoxy en C₁-C₄ ou formant avec l'atome de carbone du cycle un groupe carbonyle,
pour le traitement de la peau en vue de la protéger ou de lutter contre le photovieillissement et pour la prévention de l'acné ou la lutte contre l'acné.

Le groupe alcoxy en C₁-C₄ est de préférence un groupe méthoxy.

Le caroténoïde de formule (I) est plus particulièrement choisi parmi le β-carotène, l'α-carotène, le γ-carotène, la canthaxanthine, la lutéine, la zéaxanthine et l'astaxanthine.

Les caroténoïdes photoconvertibles de formule (I) utilisés selon l'invention sont des précurseurs de rétinoïdes qui, sous l'effet d'un "stress oxydant", libèrent du rétinol et des acides rétinoïques de façon contrôlée. Cette libération est en quelque sorte programmée par le stress et de ce fait, la composition n'agit que lorsque le besoin s'en fait sentir.

L'invention a également pour objet une composition cosmétique ou dermatologique à libération contrôlée d'actif, contenant au moins un caroténoïde apte à se transformer en rétinol et acide rétinoïque et ses isomères ayant la formule (I) ci-dessus.

La composition cosmétique ou dermatologique à libération contrôlée d'actif selon l'invention est destinée en particulier à la prévention du photovieillissement et de l'acné et/ou à la lutte contre l'acné et le photovieillissement.

Dans son application à la prévention du photovieillissement et/ou à la lutte contre le photovieillissement, la composition cosmétique ou dermatologique selon l'invention procure une sensation de confort et de douceur, permet d'atténuer les rougeurs du visage, ainsi que les rides et les ridules, en réduisant la rugosité de la peau et donne au teint éclat et uniformité, tout en étant bien tolérée.

La composition cosmétique ou dermatologique selon l'invention contient 0,0001 à 10% en poids, et de préférence 0,0001 à 5% en poids d'au moins un caroténoïde de formule (I) tel que défini ci-dessus, dans un milieu cosmétiquement ou dermatologiquement acceptable.

La composition cosmétique ou dermatologique selon l'invention peut contenir, en outre, au moins un principe actif cosmétique ou dermatologique choisi parmi les anti-inflammatoires les anti-acnéiques, les antifongiques, les antibactériens, les anti-séborrhéiques, les vitamines, les agents kératolytiques, les hydratants, les agents antiradicaux libres et les antioxydants.

La présente invention a également pour objet l'utilisation d'un caroténoïde photoconvertible de formule (I) tel que défini ci-dessus pour la fabrication d'une composition cosmétique ou dermatologique destinée à la prévention du photovieillissement ou à la lutte contre le photovieillissement de la peau ou destinée à la prévention de l'acné ou à la lutte contre l'acné.

Les compositions cosmétiques ou dermatologiques selon l'invention peuvent être formulées par exemple sous forme d'émulsion, notamment de crème, sous forme de gel, de poudre, de produit anhydre, d'émulsion huile-dans-eau formée par des globules huileux pourvus d'un enrobage cristal liquide lamellaire telle que décrite dans EP-A-0 641 557, ou encore de dispersion aqueuse de vésicules contenant des liposomes, des niosomes ou des nanocapsules. Un liposome est une vésicule dont la membrane est constituée de lipides ioniques et un "niosome" est une vésicule dont la membrane est constituée de lipides non-ioniques. Les systèmes vésiculaires sont obtenus selon des techniques classiques, par exemple selon les demandes de brevet FR-A-2 315 991, 2 408 387, 2 597 346 et 2 597 367. Les nanocapsules sont préparées à partir de polymères en émulsion, notamment selon la demande de brevet FR-A-2 659 554.

L'invention a aussi pour objet un procédé de traitement cosmétique de la peau consistant à appliquer avant exposition aux rayons ultraviolets, une quantité suffisante d'une composition cosmétique telle- que définie ci-dessus.

L'invention sera mieux illustrée à l'aide des exemples suivants.

### Exemple 1 : Crème de jour à base de liposomes

On prépare, dans une première étape, une dispersion aqueuse liposomée, comprenant :

| | |
|---|---|
| - Phytostérol oxyéthyléné à 5 moles d'oxyde d'éthylène | 1,2 g |
| - Lécithine de soja hydrogénée | 1,8 g |
| - β-carotène | 0,3 g |
| - Glycérine | 3 g |
| - Eau déminéralisée | 15 g |

Dans une deuxième étape, on ajoute à la phase liposomée la phase grasse suivante :

| | |
|---|---|
| - Huile d'abricot | 10 g |
| - Cyclodiméthicone | 5 g |

On soumet le tout à une agitation mécanique, puis on ajoute les substances suivantes :

| | |
|---|---|
| - Polymère carboxyvinylique (mélange d'acides carboxy-vinyliques commercialisé sous le nom de CARBOPOL 940 par la Société GOODRICH) | 0,4 g |
| - Conservateur | 0,3 g |
| - Triéthanolamine qs pH = 6 | |
| - Eau déminéralisée qs | 100 g |

Le β-carotène est ainsi stabilisé dans la membrane des vésicules lipidiques.

Appliquée sur le visage tous les matins pendant une période de 1 à 3 mois, cette crème permet d'atténuer les rides et ridules, confère à la peau un aspect lisse et donne un teint éclatant et uniforme.

### Exemple 2 : Crème de jour à base de "niosomes" (lipides non-ioniques)

On réalise la co-fusion des lipides suivants, sous atmosphère inerte, à 110°C :

| | |
|---|---|
| - Dicétylphosphate | 0,3 g |
| - Polyglycérolhexadécyléther | 1,35 g |
| - Cholestérol | 1,35 g |

On ramène la température du mélange fondu à 90°C.

On ajoute 0,3 g de β-carotène.

On forme alors une phase lamellaire en ajoutant la phase aqueuse suivante :

| | |
|---|---|
| - Glycérine | 3 g |
| - Eau déminéralisée | 15 g |

On homogénise le mélange à la température de 60°C.

On ajoute ensuite la phase grasse suivante à température ambiante :

| | |
|---|---|
| - Huile de vaseline | 10 g |
| - Isohexadécane | 3 g |
| - Cyclohexadiméthicone | 2 g |

On introduit dans un ultradisperseur.

On ajoute la phase gélifiée suivante :

| | |
|---|---|
| - Polymère carboxyvinylique (mélange d'acides carboxyvinyliques commercialisé sous le nom de CARBOPOL 940 par la Société GOODRICH) | 0,4 g |
| - Conservateur | 0,3 g |
| - Triéthanolamine qs pH = 6 | |
| - Eau déminéralisée qs | 100 g |

### Exemple 3 : Poudre

A partir des constituants suivants, on prépare une émulsion à 60°C :

| | |
|---|---|
| - Caséinate de sodium | 60 g |
| - Gomme de xanthane | 30 g |
| - Huile d'abricot | 198 g |
| - Vitamine E | 5 g |
| - β-carotène | 2 g |
| - Eau | 705 g |

On déshydrate l'émulsion par atomisation.

On obtient une poudre qu'on peut appliquer directement sur la peau du visage le matin.

Cette poudre confère à la peau un aspect lisse en corrigeant les rides et les ridules, permet de l'épaissir et confère un teint plus éclatant.

### Exemple 4 : Produit anhydre

On prépare la composition suivante :

| | |
|---|---|
| - Huile de vaseline | 14,5 g |
| - Cire microcristalline | 30 g |
| - Charge de polyéthylène | 15 g |
| - Huile d'abricot | 5 g |
| - Beurre de karité | 20 g |
| - β-carotène | 0,5 g |
| - Cire de camauba | 15 g |

Le milieu anhydre confère au β-carotène sa stabilité.

Ce produit, appliqué sur la peau du visage tous les matins pendant 1 à 3 mois, atténue les rides et les ridules en lui conférant un aspect lisse et donne un teint plus éclatant.

## Revendications

1. Utilisation d'un caroténoïde photoconvertible, apte à se transformer en rétinol et acide rétinoïque ou ses isomères, de formule : 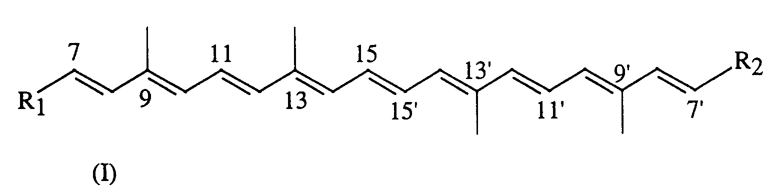 dans laquelle les substituants R₁ et R₂ désignent l'un des groupements suivants : 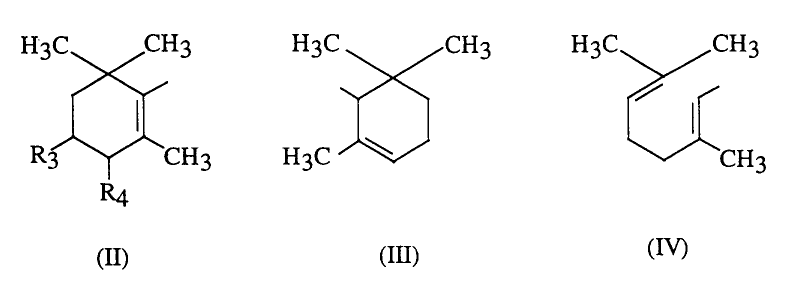 l'un au moins des substituants R₁ ou R₂ désignant un groupement β-ionone de formule (II),
les substituants R₃ et R₄ désignant de l'hydrogène, un groupe hydroxy, carboxy, alcoxy en C₁-C₄, de préférence méthoxy ou formant avec l'atome de carbone du cycle un groupe carbonyle,
pour le traitement cosmétique topique de la peau en vue de la protéger contre le photovieillissement.

2. Utilisation d'un caroténoïde photoconvertible de formule (I) selon la revendication 1 choisi parmi le β-carotène, l'α-carotène, le γ-carotène, la canthaxanthine, la lutéine, la zéaxanthine et l'astaxanthine pour le traitement cosmétique de la peau en vue de la protéger contre le photovieillissement.

3. Composition cosmétique ou dermatologique topique à libération contrôlée d'actif, caractérisée par le fait qu'elle contient au moins un caroténoïde photoconvertible, apte à se transformer en rétinol et acide rétinoïque ou ses isomères, de formule (I) suivante : 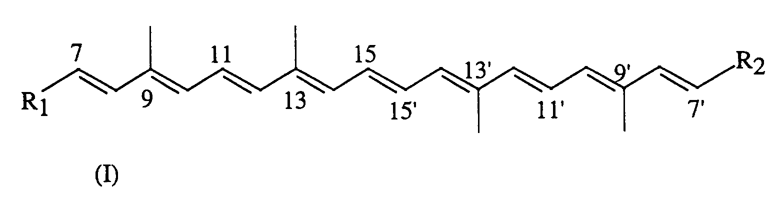 dans laquelle les substituants R₁ et R₂ désignent l'un des groupements suivants : 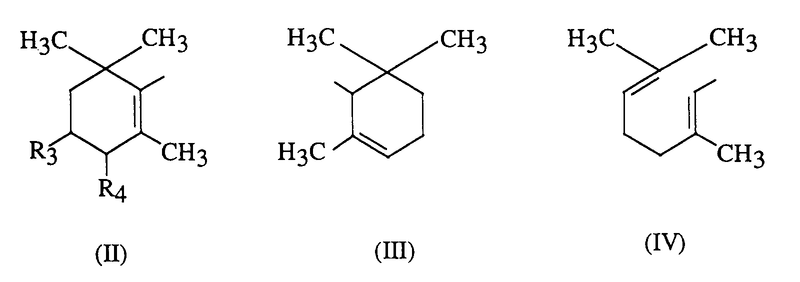 l'un au moins des substituants R₁ ou R₂ désignant un groupement β-ionone de formule (II),
les substituants R₃ et R₄ désignant de l'hydrogène, un groupe hydroxy, carboxy, alcoxy en C₁-C₄, de préférence méthoxy ou formant avec l'atome de carbone du cycle un groupe carbonyle,
dans un milieu cosmétiquement ou dermatologiquement acceptable.

4. Composition selon la revendication 3, caractérisée par le fait qu'elle contient 0,0001 à 10% en poids, et de préférence 0,0001 à 5% en poids d'au moins un composé de formule (I) tel que défini dans la revendication 3, dans un milieu cosmétiquement ou dermatologiquement acceptable.

5. Composition selon la revendication 3 ou 4, caractérisée par le fait qu'elle comprend au moins un composé de formule (I) choisi parmi le β-carotène, l'α-carotène, le γ-carotène, la canthaxanthine, la lutéine, la zéaxanthine et l'astaxanthine.

6. Composition cosmétique ou dermatologique selon l'une quelconque des revendications 3 à 5, caractérisée par le fait qu'elle contient, en outre, au moins un principe actif cosmétique ou dermatologique, choisi parmi les anti-inflammatoires, les anti-acnéiques, les antifongiques, les antibactériens, les antiséborrhéiques, les vitamines, les agents kératolytiques, les hydratants, les agents antiradicaux libres et les anti-oxydants.

7. Composition cosmétique ou dermatologique selon l'une quelconque des revendications 3 à 6, caractérisée par le fait qu'elle est sous forme d'émulsion, de gel, de dispersion aqueuse de vésicules, de poudre ou de produit anhydre.

8. Composition cosmétique selon l'une quelconque des revendications 3 à 7, pour son utilisation pour la prévention du photovieillissement ou pour la lutte contre le photovieillissement de la peau.

9. Composition cosmétique selon l'une quelconque des revendications 3 à 8, pour son utilisation pour l'atténuation des rides et des ridules.

10. Composition dermatologique selon l'une quelconque des revendications 3 à 7, pour son utilisation pour la prévention de l'acné ou pour la lutte contre l'acné.

11. Utilisation d'un caroténoïde photoconvertible de formule (I) selon la revendication 3, pour la fabrication d'une composition dermatologique topique destinée à la prévention de l'acné ou à la lutte contre l'acné.

12. Utilisation d'un caroténoïde photoconvertible de formule (I) selon la revendication 3, pour la préparation d'une composition cosmétique topique destinée à la prévention du photovieillissement ou à la lune contre le photovieillissement de la peau.

13. Procédé de traitement cosmétique de la peau, caractérisé par le fait qu'il consiste à appliquer sur la peau, avant exposition aux rayons ultraviolets, une quantité suffisante d'une composition cosmétique selon l'une quelconque des revendications 3 à 9.

## Claims

1. Use of a photoconvertible carotenoid, capable of being converted to retinol and retinoic acid or its isomers, of formula: 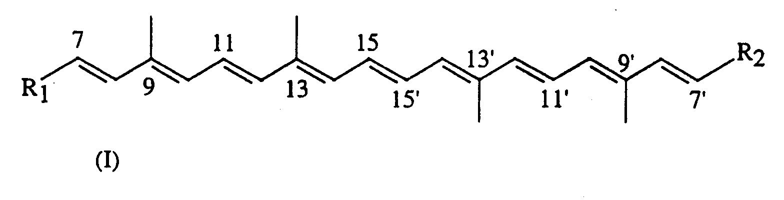 in which the R₁ and R₂ substituents denote one of the following groups: 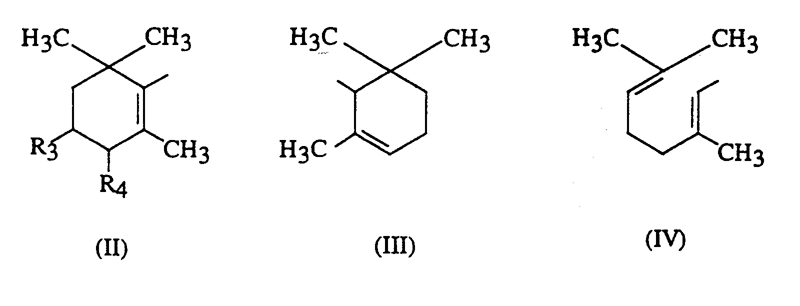 at least one of the R₁ or [sic] R₂ substituents denoting a β-ionone group of formula (II),
the R₃ and R₄ substituents denoting hydrogen, a hydroxyl, carboxyl or C₁-C₄ alkoxy, preferably methoxy, group or forming, with the carbon atom of the ring, a carbonyl group,
for the topical cosmetic treatment of the skin for the purpose of protecting it against photoaging.

2. Use of a photoconvertible carotenoid of formula (I) according to Claim 1 chosen from β-carotene, α-carotene, γ-carotene, canthaxanthin, lutein, zeaxanthin and astaxanthin for the cosmetic treatment of the skin for the purpose of protecting it against photoaging.

3. Topical cosmetic or dermatological composition with controlled release of active principle, characterized in that it contains at least one photoconvertible carotenoid, capable of being converted to retinol and retinoic acid or its isomers, of following formula (I): 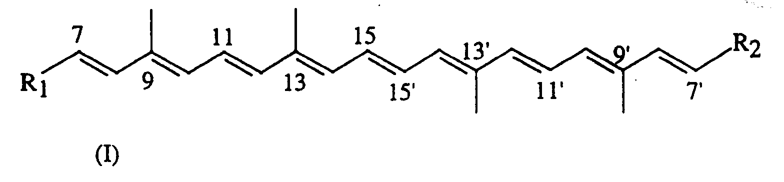 in which the R₁ and R₂ substituents denote one of the following groups: 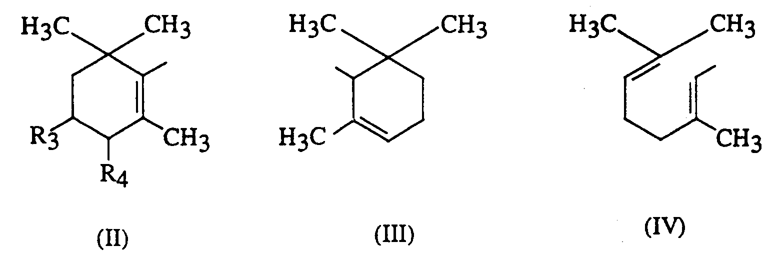 at least one of the R₁ or [sic] R₂ substituents denoting a β-ionone group of formula (II),
the R₃ and R₄ substituents denoting hydrogen, a hydroxyl, carboxyl or C₁-C₄ alkoxy, preferably methoxy, group or forming, with the carbon atom of the ring, a carbonyl group,
in a cosmetically or dermatologically acceptable medium.

4. Composition according to Claim 3, characterized in that it contains 0.0001 to 10% by weight, and preferably 0.0001 to 5% by weight, of at least one compound of formula (I) as defined in Claim 3, in a cosmetically or dermatologically acceptable medium.

5. Composition according to Claim 3 or 4, characterized in that it comprises at least one compound of formula (I) chosen from β-carotene, α-carotene, γ-carotene, canthaxanthin, lutein, zeaxanthin and astaxanthin.

6. Cosmetic or dermatological composition according to any one of Claims 3 to 5, characterized in chat it additionally contains at least one cosmetic or dermatological active principle chosen from anti-inflammatories, anti-acne agents, antifungals, anti-bacterials, antiseborrhoeic agents, vitamins, keratolytic agents, humectants, agents for combating free radicals and antioxidants.

7. Cosmetic or dermatological composition according to any one of Claims 3 to 6, characterized in that it is in the form of an emulsion, of a gel, of an aqueous dispersion of vesicles, of a powder or of an anhydrous product.

8. Cosmetic composition according to any one of Claims 3 to 7, for its use in the prevention of photoaging or in the control of photoaging of the skin.

9. Cosmetic composition according to any one of Claims 3 to 8, for its use in toning down wrinkles and fine lines.

10. Dermatological composition according to any one of Claims 3 to 7, for its use in the prevention of acne or in the control of acne.

11. Use of a photoconvertible carotenoid of formula (I) according to Claim 3, for the manufacture of a topical dermatological composition intended for the prevention of acne or for the control of acne.

12. Use of a photoconvertible carotenoid of formula (I) according to Claim 3, for the preparation of a topical cosmetic composition intended for the prevention of photoaging or for the control of photoaging of the skin.

13. Process for the cosmetic treatment of the skin, characterized in that it comprises the application to the skin, before exposure to ultraviolet radiation, of a sufficient amount of a cosmetic composition according to any one of Claims 3 to 9.

## Patentansprüche

1. Verwendung eines fotoumsetzbaren Karotinoids, das geeignet ist, in Retinol und Retinsäure oder deren Isomeren überführt zu werden, und die Formel aufweist: 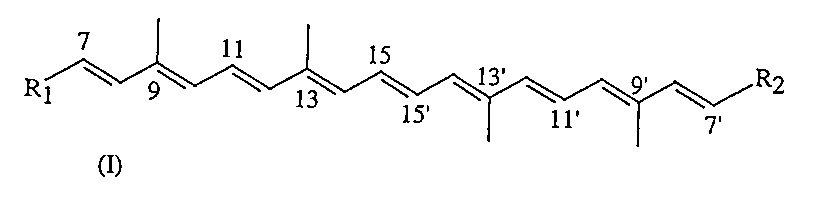 worin die Substituenten R₁ nd R₂ eine der folgenden Gruppierungen bedeuten: 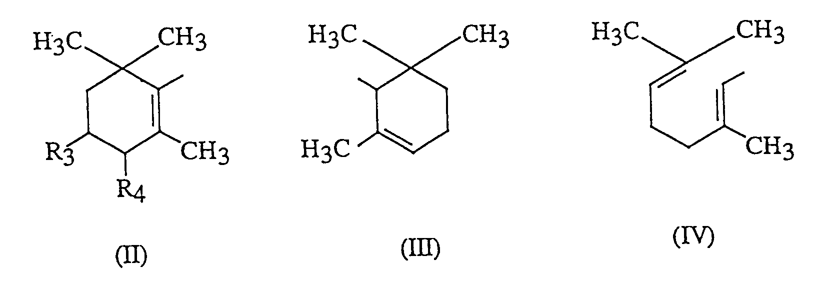 wobei mindestens einer der Substituenten R₁ oder R₂ eine β-Ionon-Gruppierung der Formel (II) bedeutet,
und worin die Substituenten R₃ und R₄ Wasserstoff, eine Hydroxy-, Carboxy- und C₁₋₄-Alkoxy- und vorzugsweise eine Methoxygruppe bedeuten oder mit dem Kohlenstoffatom eines gebildeten Rings eine Carbonylgruppe bilden,
zur kosmetischen topischen Behandlung der Haut, um sie vor Fotoalterung zu schützen.

2. Verwendung gemäß Anspruch 1 eines fotoumsetzbaren Karotinoids der Formel (I), ausgewählt aus β-Karotin, α-Karotin, γ-Karotin, Kanthaxanthin, Lutein, Zeaxanthin und aus Astaxanthin, zur kosmetischen Behandlung der Haut, um sie vor Fotoalterung zu schützen.

3. Kosmetische oder dermatologische topische Zusammensetzung zur gesteuerten Freisetzung eines Wirkstoffs,
dadurch **gekennzeichnet**, daß
sie mindestens ein fotoumsetzbares Karotinoid, das sich dazu eignet, in Retinol und Retinsäure oder deren Isomeren überführt zu werden, und die folgende Formel (I) aufweist: 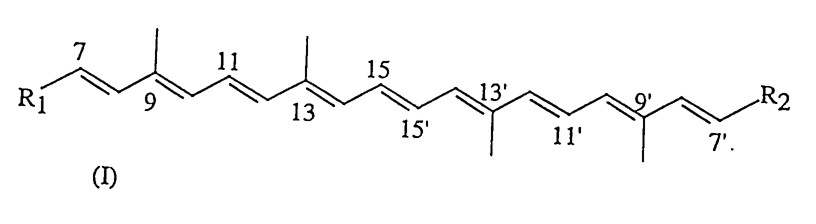 worin die Substituenten R₁ und R₂ eine der folgenden Gruppierungen bedeuten: 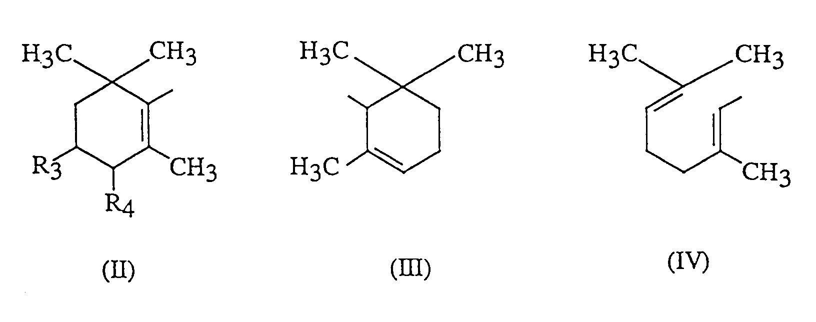 wobei mindestens einer der Substituenten R₁ oder R₂ eine β-Ionon-Gruppierung der Formel (II) bedeutet,
und worin die Substituenten R₃ und R₄ Wasserstoff, eine Hydroxy-, Carboxy- und eine C₁₋₄-Alkoxy- und vorzugsweise eine Methoxygruppe bedeuten oder mit dem Kohlenstoffatom eines gebildeten Rings eine Carbonylgruppe bilden,
in einem kosmetisch oder dermatologisch geeigneten Milieu enthält.

4. Zusammensetzung gemäß Anspruch 1,
dadurch **gekennzeichnet**, daß
sie 0,0001 bis 10 und vorzugsweise 0,0001 bis 5 Gew.% mindestens einer der in Anspruch 3 definierten Verbindung der Formel (I) in einem kosmetisch oder dermatologisch geeigneten Milieu enthält.

5. Zusammensetzung gemäß Anspruch 3 oder 4,
dadurch **gekennzeichnet**, daß
sie mindestens eine Verbindung der Formel (I) enthält, ausgewählt aus β-Karotin, α-Karotin, γ-Karotin, Kanthaxanthin, Luthein, Zeaxanthin und aus Astaxanthin.

6. Kosmetische oder dermatologische Zusammensetzung gemäß einem der Ansprüche 3 bis 5,
dadurch **gekennzeichnet**, daß
sie, ausserdem, mindestens ein kosmetisches oder dermatologisches Wirkstoffprinzip enthält, ausgewählt aus entzündungshemmenden Mitteln, Antiaknemitteln, Antipilzmitteln, antibakteriellen Mitteln, antiseborrheischen Mitteln, Vitaminen, keratolytischen Mitteln, Hydratisiermitteln, Mitteln gegen freie Radikale und aus Antioxidantien.

7. Kosmetische oder dermatologische Zusammensetzung gemäß einem der Ansprüche 3 bis 6,
dadurch **gekennzeichnet**, daß
sie in Form einer Emulsion, eines Gels, einer wässrigen Dispersion aus Bläschen, eines Pulvers oder eines wasserfreien Produkts vorliegt.

8. Kosmetische Zusammensetzung gemäß einem der Ansprüche 3 bis 7 zur Verwendung zur Vorbeugung der Fotoalterung oder zur Bekämpfung der Fotoalterung der Haut.

9. Kosmetische Zusammensetzung gemäß einem der Ansprüche 3 bis 8 zur Verwendung zur Milderung von Falten und Fältchen.

10. Dermatologische Zusammensetzung gemäß einem der Ansprüche 3 bis 7 zur Verwendung zur Vorbeugung von Akne oder zur Bekämpfung von Akne.

11. Verwendung gemäß Anspruch 3 eines fotoumsetzbaren Karotinoids der Formel (I) zur Herstellung einer dermatologischen topischen Zusammensetzung zur Vorbeugung von Akne oder zur Bekämpfung von Akne.

12. Verwendung gemäß Anspruch 3 eines fotoumsetzbaren Karotinoids der Formel (I) zur Zubereitung einer kosmetischen topischen Zusammensetzung zur Vorbeugung der Fotoalterung oder Bekämpfung der Fotoalterung der Haut.

13. Verfahren zur kosmetischen Behandlung der Haut,
dadurch **gekennzeichnet**, daß
man auf die Haut, vor Aussetzen auf ultraviolette Strahlen, eine hinreichende Menge einer kosmetischen Zusammensetzung gemäß eines jeden der Ansprüche 3 bis 9 aufträgt und zur Anwendung gelangen läßt.
